# FASCICULE DE BREVET EUROPEEN

(11) **EP 1 015 481 B1**
(45) Date de publication et mention de la délivrance du brevet: **03.05.2006**
(21) Numéro de dépôt: 98913842.5
(22) Date de dépôt: 05.03.1998
(51) Int. Cl.: C07K 14/18, C07K 16/10, A61K 39/12, G01N 33/569, A61K 38/16, C12Q 1/70

(54) **PEPTIDE STRUCTURAL ANTIGENIQUE, COMPOSES ANTIGENIQUE ET IMMUNOGENE, ET UTILISATIONS DANS LA DETECTION, LA PREVENTION ET LE TRAITEMENT D'UNE INFECTION PAR LE VHC**
STRUKTURELLES PEPTID MIT ANTIGENISCHENEN EIGENSCHAFTEN, ANTIGENISCHEN VERBINDUNGEN UND VERWENDUNGEN IM NACHWEIS, VORBEUGUNG UND BEHANDLUNG EINER VHC-INFEKTION
ANTIGENIC STRUCTURAL PEPTIDE, ANTIGENIC AND IMMUNOGENIC COMPOUNDS AND USES FOR DETECTING, PREVENTING AND TREATING AN HCV INFECTION

(30) Priorité: 05.03.1997 FR 9702878
(43) Date de publication de la demande: 05.07.2000
(73) Titulaire: BIO MERIEUX, 69280 Marcy l'Etoile (FR)
(72) Inventeur: JOLIVET, Michel, F-69500 Bron (FR); PENIN, François, F-69150 Décines (FR); DALBON, Pascal, F-69006 Lyon (FR); LADAVIERE, Laurent, F-69100 Villeurbanne (FR); LACOUX, Xavier, F-69008 Lyon (FR)
(74) Mandataire: Guerre, Dominique
(86) Numéro de dépôt international: PCT/FR1998/000442
(87) Numéro de publication internationale: WO 1998/039360

(56) Documents cités:
- EP-A- 0 388 232
- EP-A- 0 569 309
- EP-A- 0 628 572
- DATABASE WPI Week 97 Derwent Publications Ltd., London, GB; AN 95-047903 XP002048544 "Detection of hepatitis V virus using oligopeptide fragment of HCV core region" & JP 06 327 482 A (IMMUNO JAPAN KK) , 29 novembre 1994
- CHEMICAL ABSTRACTS, vol. 119, no. 5, 2 août 1993 Columbus, Ohio, US; abstract no. 47025, XP002048543 & T SUGIYASU: "Enzyme-linked immunosorbent assay for antibodies against core protein of hepatitis C virus with a synthetic oligopeptide (N14E plus #19-#40)" FUKUOKA IGAKU ZASSHI, vol. 84, no. 4, 1993, pages 148-150,
- W-M CHING ET AL.: "Interaction of immune sera with synthetic peptides corresponding to the structural protein region of hepatitis C virus" PROCEEDINGS OF THE NATIONAL ACADEMY OF SCIENCES OF USA, vol. 89, avril 1992, WASHINGTON US, pages 3190-3194, XP002048541
- A CERINO ET AL.: "A human monoclonal antibody specific for the N terminus of the hepatitis C virus nucleocapsid protein" JOURNAL OF IMMUNOLOGY, vol. 151, no. 12, 15 décembre 1993, BALTIMORE US, pages 7005-7015, XP002048542

## Description

La présente invention concerne la caractérisation d'un site antigénique appartenant à une région pluriépitopique immunodominante, localisée dans l'extrémité N-terminale de la protéine Core ou nucléocapside (ou protéine p21) du virus de l'hépatite C (VHC), [B. Hosein et al., Proc. Natl. Acad. Sci. USA, 88, 3647-51 (1991)] ainsi que les applications qui en découlent, notamment dans le diagnostic, le traitement ou la prévention d'une infection par le VHC.

Conformément au document EP-A-0 569 309, au nom de la Demanderesse, on connaît un peptide s'étendant depuis l'acide aminé en position 2 jusqu'à l'acide aminé en position 45 de l'extrémité N-terminale de la protéine de la nucléocapside du VHC, et dont la séquence est identifiée par SEQ ID NO: 1. Ce peptide détermine une région immunodominante qui suffit, à elle-seule, pour obtenir les mêmes sensibilité et spécificité en termes de détection des anticorps dirigés contre le VHC, qu'avec la protéine de la nucléocapside dans son entier.

En poursuivant ces travaux sur le peptide défini précédemment, la Demanderesse a découvert que :
- la région s'étendant depuis l'acide aminé 15 jusqu'à l'acide aminé 39 de l'extrémité N-terminale de la protéine de la nucléocapside définit la localisation d'un épitope immunodominant,
- cet épitope immunodominant est de type conformationnel, et s'identifie à un motif tridimensionnel de type hélice-boucle ou coude-hélice, dans lequel les deux hélices sont disposées sensiblement perpendiculairement l'une par rapport à l'autre.

Ainsi, l'invention concerne un peptide structural, reconnu par les anticorps dirigés contre un polypeptide comprenant la séquence des acides aminés 2-45 de l'extrémité N-terminale de la protéine Core (p21) du virus de l'hépatite C (VHC) ou de tout variant dudit virus, et qui comprend, ou consiste en, une structure tertiaire constituée par au moins un premier fragment peptidique ayant une structure secondaire en hélice α, un second fragment peptidique ayant une structure secondaire en hélice α et un troisième fragment peptidique de jonction reliant les deux hélices α, ces deux hélices α étant sensiblement perpendiculaires l'une par rapport à l'autre, dans l'espace. L'angle formé par les deux hélices α est avantageusement de 90° ± 10°, ledit peptide consistant en la séquence SEQ ID NO : 2 ou en une séquence peptidique équivalente à SEQ ID NO :2 choisie parmi l'une quelconque des séquences SEQ ID NO :3 à SEQ ID NO :10.

Le troisième fragment peptidique de jonction est de préférence choisi parmi les boucles et les structures secondaires en coudes notamment les coudes β.

Selon la présente invention, sont exclus de la définition précédente, et des définitions suivantes, tous composés protéiques ou peptidiques, comprenant ou constitués par l'extrémité N-terminale de la protéine p21, cette extrémité étant considérée comme débutant à l'acide aminé en position 1 ou l'acide aminé en position 2.

Avant de décrire la présente invention plus en détails et d'en livrer l'intérêt et les applications, une définition de termes employés dans la description et les revendications est ci-après donnée.

Par épitope conformationnel, on entend une région protéique, reconnue par des anticorps et déterminée notamment par des acides aminés qui peuvent être éloignés dans la séquence peptidique, mais qui, du fait du repliement de la chaîne polypeptidique, se retrouvent proches les uns des autres dans l'espace, et peuvent ainsi former un motif susceptible d'être reconnu par l'anticorps.

Par peptide, on désigne un enchaînement d'acides aminés, obtenu par synthèse chimique ou par des techniques de recombinaison génétique. Les peptides selon l'invention peuvent être obtenus par des méthodes de synthèse classique, par exemple avec un synthétiseur automatique de peptides, ou par les techniques de génie génétique comprenant l'insertion d'une séquence d'ADN codant pour ledit peptide dans un vecteur d'expression tel qu'un plasmide ou un virus, et la transformation de cellules eucaryotes ou procaryotes avec ce vecteur d'expression et culture de ces cellules.

Par séquence peptidique équivalente à une séquence peptidique de référence (telle que SEQ ID NO: 2 dans le cas de la présente invention), ladite séquence peptidique de référence ayant un motif de référence, on entend une séquence d'acides aminés modifiée par insertion et/ou délétion et/ou substitution et/ou allongement et/ou raccourcissement et/ou modification chimique d'un ou plusieurs acides aminés, pour autant que ces modifications, d'une part, préservent substantiellement, voire amplifient, les propriétés antigéniques de ladite séquence peptidique de référence, et, d'autre part, conservent essentiellement la structure tertiaire de référence de ladite séquence peptidique de référence.

Ainsi, on entend par "séquence équivalente", notamment les séquences dans lesquelles un ou plusieurs acides aminés sont substitués par un ou plusieurs autres acides aminés ; les séquences dans lesquelles un ou plusieurs acides aminés de la série L sont remplacés par un ou plusieurs acides aminés de la série D ; les séquences dans lesquelles on a introduit une modification des chaînes latérales des acides aminés, telle qu'une acétylation des fonctions amines, une carboxylation des fonctions thiols, une estérification des fonctions carboxyliques, une substitution d'un atome d'oxygène par un atome de soufre, ou autres ; une modification des liaisons peptidiques telles que des liaisons carba, rétro, inverso, rétro-inverso, réduites et méthylène-oxy.

On comprend dans les séquences peptidiques équivalentes à SEQ ID NO: 2, celles de peptides mimotopes qui ont une séquence peptidique peu différente de SEQ ID NO: 2, tout en possédant la même structure tertiaire et les mêmes propriétés antigéniques que SEQ ID NO: 2, ces peptides mimotopes sont choisis parmi les séquences peptidiques SEQ ID N°3 à 10.

La structure secondaire d'un fragment peptidique se définit comme une structure tridimensionnelle, stabilisée essentiellement par des liaisons hydrogène impliquant les atomes qui construisent la structure primaire. On distingue plusieurs types de structures secondaires, et en particulier la structure en hélice α, la structure en coude β.

L'hélice α a été décrite par Pauling et al. [L. Pauling, Proc. Natl. Acad. Sci. USA, 37, 205-211 (1951)], et correspond à une hélice de pas droit. L'hélice a est stabilisée essentiellement par des liaisons hydrogènes entre l'oxygène du groupement carbonyle de l'acide aminé en position i et l'hydrogène du groupement amide de l'acide aminé en position i+4. Ces liaisons hydrogènes sont presque parallèles à l'axe longitudinal de l'hélice, et les N, H et O impliqués dans la liaison hydrogène sont pratiquement colinéaires. Les résidus d'acides aminés voisins sont distants de 1,5 Å sur l'axe de l'hélice α, et un tour complet correspond à 3,6 résidus d'acides aminés. Le déplacement le long de l'axe de l'hélice est donc de 5,4 Å par tour.

Les paramètres de la structure en hélice α sont les suivants :
Angles en degrés :
   Φ -57
   Ψ -47
   ω 180
Acides aminés par tour 3,6,
Distance inter résidus en Å (C_{αi}-c_{αi+1})- 1,5

Les coudes β sont considérés comme une structure secondaire dont au moins 7 types différents ont été décrits [P. Chou et G.D. Fasman, J. Mol. Biol., 115, 135-175 (1977)]. Ils permettent le repliement de la chaîne polypeptidique et impliquent au minimum 4 acides aminés. Ils permettent généralement un changement de direction entre 2 structures secondaires.

Enfin, les boucles qui ne sont généralement pas considérées comme une structure secondaire, correspondent à un segment continu de chaîne polypeptidique décrivant le plus souvent la lettre Ω dans l'espace d'où leur nom de boucle oméga [J. Leszczinski et J. Rose, Sciences, 234, 849-855 (1986)]. Une boucle est définie par sa longueur et une faible distance entre ses extrémités. Les boucles sont stabilisées et définies par des interactions au niveau des chaînes latérales de leurs résidus constitutifs.

Pour obtenir un motif, ou structure tertiaire de référence répondant à la définition précédente selon l'invention, celui-ci comprend de préférence une structure primaire équivalente aux acides aminés s'étendant depuis l'acide aminé en position 15 jusqu'à l'acide aminé en position 39 de l'extrémité N-terminale de la protéine de la nucléocapside du virus de l'hépatite C ou de la protéine de la nucléocapside d'un variant dudit virus.

Plus avantageusement, cette structure primaire comprend, ou consiste en toute séquence peptidique équivalente à SEQ ID NO: 2 telle que définie précédemment. Quand le peptide structural a la structure primaire identifié par SEQ ID NO: 2, le premier fragment peptidiqué ayant une structure secondaire en hélice α est constitué par la séquence Thr Asn Arg Arg Pro Gln Asp Val Lys Phe (1 à 10), le second fragment peptidique ayant une structure secondaire en hélice α est constituée par la séquence Gln Ile Val Cly Gly Val Tyr Leu Leu Pro Arg (15 à 25), et le troisième fragment peptidique est constitué par la séquence Pro Gly Gly Gly (11 à 14).

L'invention concerne aussi un composé antigénique, reconnu par les anticorps dirigés contre un polypeptide comprenant, ou constitué par, la séquence des acides aminés 2-45 de l'extrémité N-terminale de la protéine core (p21) du virus de l'hépatite C (VHC) ou de tout variant dudit virus, ainsi qu'un composé immunogène, susceptible d'induire la production d'anticorps, ledit composé antigénique ou immunogène comprenant un peptide selon l'invention à l'exclusion de tous composés protéiques ou peptidiques, comprenant ou constitués par l'extrémité N-terminale de la protéine p21, cette extrémité étant considérée comme débutant à l'acide aminé en position 1 ou l'acide aminé en position 2- A titre d'exemples, le peptide selon l'invention peut être conjugué selon des techniques bien connues de l'homme du métier, à une molécule porteuse comme par exemple une protéine naturelle ou recombinante, un polymère synthétique d'acides aminés ou à chaînes aliphatiques, un fragment nucléique ou une molécule traceuse ou marqueur comme par exemple un oligonucléotide, une enzyme telle que la peroxydase de raifort, la phosphatase alcaline ou la β-galactosidase ou encore un radioélément. Le peptide de l'invention peut être fixé sur tout support.

Les objets de l'invention décrits ci-dessus, à savoir peptide et composé antigénique ou immunogène sont utilisables pour détecter et/ou quantifier des anticorps dirigés contre la p21 du VHC ou de tout variant dudit virus, et les anticorps précités, peuvent être utilisés pour détecter et/ou quantifier les antigènes du vHC ou de tout variant dudit virus.

Ces objets peuvent en outre être appliqués à la préparation d'une composition diagnostique, prophylactique ou thérapeutique, destinée à détecter, prévenir ou traiter une infection par le VHC ou de tout variant dudit virus.

C'est pourquoi l'invention concerne aussi une composition pharmaceutique comprenant un motif, un peptide, un composé, définis ci-dessus.

L'invention concerne en outre :
- un procédé de détection et/ou quantification, dans un échantillon, soit d'anticorps dirigés contre la protéine de la nucléocapside du VHC, soit d'antigènes du VHC ou d'un variant dudit virus, comprenant les étapes consistant à mettre en contact ledit échantillon avec, soit un peptide et/ou un composé définis ci-dessus, et à détecter la formation d'un complexe immun, soit entre lesdits anticorps et ledit peptide ou composé de l'invention;
- un procédé de détection et/ou quantification, dans un échantillon, de tout ou partie de l'ARN du VHC, comprenant les étapes consistant à mettre en contact ledit échantillon avec un peptide, et/ou un composé de l'invention, et à détecter la formation d'un complexe entre ledit ARN et ledit peptide ou composé.

L'invention concerne en outre un complexe comprenant un peptide de l'invention, et une structure moléculaire liée spécifiquement avec ledit peptide, qui peut par exemple être sélectionnée parmi des fragments peptidiques, des fragments nucléotidiques, des molécules chimiques telles que celles du type aromatique fonctionnalisé. La complexation d'un peptide de l'invention qui présente une forte affinité avec l'ARN viral notamment, peut empêcher la fixation de cet ARN et l'expression de ce dernier. Ce complexe présente donc une application dans le traitement d'une infection par le VHC.

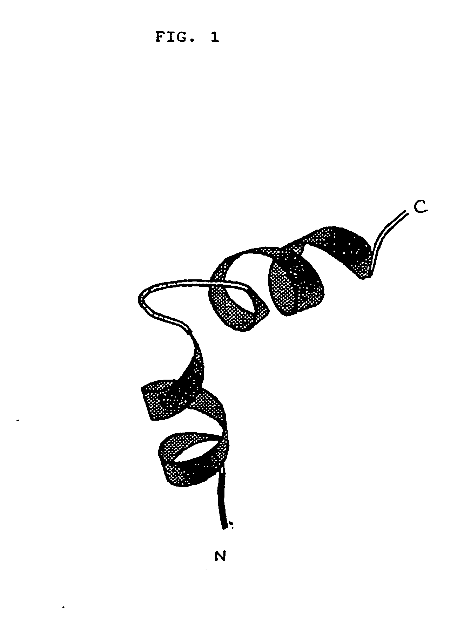

L'invention et ses caractéristiques sont détaillées dans les exemples qui suivent, et les figures 1 à 7.
La figure 1 est une représentation schématique à l'aide du logiciel Molscript de la structure tridimensionnelle du peptide de SEQ ID NO: 2, en solution dans le mélange (v/v) 40 % de 2,2,2,-trifluoroéthanol (TFE) / 60 % de tampon : NaCl 100 mM, phosphate de sodium 10 mM, à pH 5,9.
Les figures 2 et 3 représentent les spectres RMN du peptide Capsid (15-41) discutés à l'exemple 3.
La figure 4 représente la superposition de 8 structures entre l'Arg 18 et la Phe 24 ; le RMSD de la chaîne principale entre ces résidus est de 0,29 Å.
La figure 5 représente la superposition des 8 mêmes structures que pour la figure 4, entre la Glu 29 et la Leu 37 ; le RMSD de la chaîne principale entre ces résidus est de 0, 39 Å.
Les figures 6 et 7 correspondent à la superposition de 8 structures entre l'Arg 18 et la Leu 37, représentée sous deux angles différents ; le RMSD de la chaîne principale entre ces résidus est de 0,64 Å.

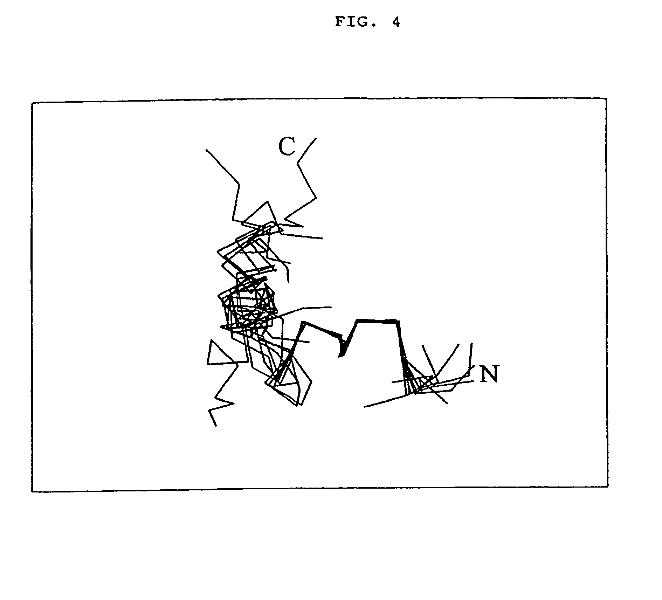

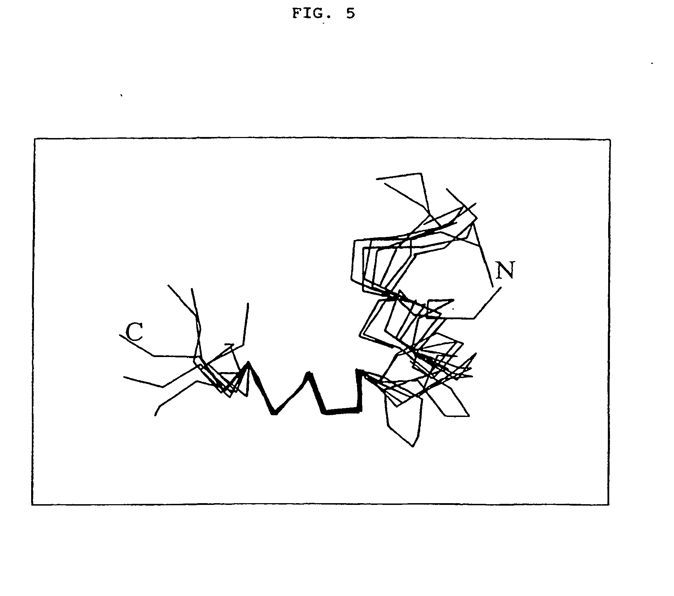

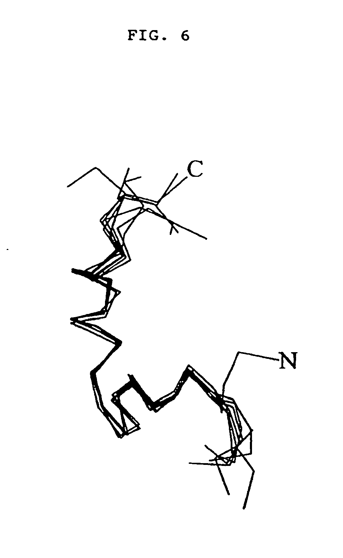

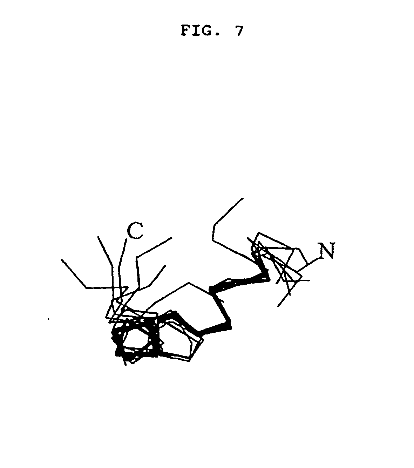

Les figures 4 à 7 sont discutées à l'exemple 4.

### EXEMPLE 1 : ANALYSE STRUCTURALE DU PEPTIDE SEQ ID NO: 2, PAR RMN ET MODELISATION MOLECULAIRE

### a) Recherche d'identité de séquences

On a essayé de savoir si le peptide étudié ne possédait pas des homologies de séquences avec des protéines dont la structure avait déjà été déterminée et répertoriée dans la banque de données PDB (Protein Data Bank). On estime généralement que lorsque deux séquences polypeptidiques ont plus de 30 % d'homologie entre elles, elles possèdent des structures 3D très similaires. Cependant, ces analyses effectuées grâce au logiciel de recherche FASTA 2.03 [D.J. Lipman et W.R. Pearson, Sciences, 227, 1435-1440 (1985)], montrent qu'il n'existe aucune séquence de structure 3D connue suffisamment homologue au peptide étudié pour envisager une modélisation moléculaire par homologie.

### b) Conditions de solvatation du peptide

Le peptide a été analysé en le plaçant dans une solution dans 40 % de 2,2,2,-trifluoroéthanol (TFE) / 60 % d' H₂O, NaCl 0,1 M, phosphate de sodium 10 mM, à pH 5, 9 et à 20°C. Ces conditions permettent de stabiliser les structures secondaires du peptide tout en conservant la structure tertiaire.

### c) Conditions d'acquisition des spectres RMN

L'ensemble des expériences RMN a été réalisé sur un spectromètre VARIAN Unity+, 500 MHz, équipé d'ultra-shims et à l'aide d'une sonde triple accord proton-carbone-azote de 5mm, équipée de gradient Z. L'enregistrement des données a été réalisé à l'aide du logiciel VNMR (version 5.1, commercialisé par VARIAN) sur une station de travail SUN IPX. Les séquences d'impulsions utilisées sont COSY, TOCSY, NOESY et ROESY. Les expériences RMN ont été enregistrées en mode phasé selon la méthode States-TPPI [D. Marion et al., J. Magn. Res., 85, 393-399 (1989)].

Les expériences ont été réalisées avec une largeur spectrale de 5500 Hz dans les deux dimensions, 2048 points de données dans la dimension t2 et 512 incréments en t1, chaque incrément étant accumulé 32 ou 64 fois. Pour les expériences DQF-COSY 4K points de données et 512 incréments ont été enregistrés. Une présaturation de faible puissance à la fréquence de l'eau, appliquée pendant le temps de relaxation d'une durée de 1,5 s (et pendant le temps de mélange dans les expériences NOESY), a été généralement utilisée pour éliminer la résonance du solvant. Pour les expériences TOCSY, le mélange isotrope est une séquence MLEV-17 pour laquelle un délai Δ égal à 2,6 fois la durée de l'impulsion à 90°, est inséré entre les impulsions [clean-TOCSY ; C. Griesinger et al., J. Am. Chem. Soc., 110, 7870-7872 (1988)]. Un *trim-pulse* de 2 ms précède la période de mélange dont la durée est fixée généralement à 80 ms. Pour les expériences ROESY, le *spin-lock* est réalisé par une irradiation continue de puissance comprise entre 3 et 4 kHz. Deux impulsions à 90° sont placées de part et d'autre de la période de mélange (ROESY compensée).

### d) Modélisation moléculaire

L'ensemble des étapes de modélisation moléculaire a été réalisé en suivant les procédures standards du programme X-plor version 3.1 [A.T. Brünger, in X-plor, A System for Crystallography and NMR, Yale University press, New Haven (1992)] sur des stations IBM rs6000 modèle 560 ou SUN Sparc 20.

La génération de la topologie consiste à utiliser une série d'informations théoriques (description des atomes, attribution des liaisons covalentes, des angles de valence) pour modéliser un peptide possédant une géométrie idéale. L'étape suivante utilise la méthode de géométrie des distances. Les intervalles de distance entre protons déterminés par RMN et les intervalles de distances théoriques liés à la structure primaire (longueur des liaisons, distances dues à la rigidité des cycles aromatiques, etc.) sont rassemblés. Il y a alors un tirage aléatoire d'un jeu de distances dans les intervalles de distances autorisés. Ces distances sont ensuite converties en coordonnées cartésiennes pour chaque atome de la molécule. Une étape d'optimisation est appliquée ; elle consiste à minimiser une fonction cible qui représente les erreurs sur les distances expérimentales observées dans les structures générées. En raison du tirage aléatoire du jeu de distance, on ne génère pas une seule structure mais une famille de structures afin d'explorer le plus largement possible tout l'espace conformationnel. Ces structures satisfont grossièrement les contraintes de distances expérimentales, cependant il existe encore souvent des imperfections dans leur géométrie covalente et leur énergie potentielle est généralement élevée. Une régularisation des structures par dynamique moléculaire avec recuit simulé ("simulated annealing") est alors appliquée. Elle consiste en une augmentation progressive de la température jusqu'à 2000°K pendant 9 picosecondes, par pas de 50°K, puis en une équilibration pendant 12 picosecondes et un refroidissement à 250°K par pas de 25°K pendant 10 picosecondes. Une seconde étape d'affinement des structures par recuit simulé est réalisée à 1000°K pendant 50 picosecondes, suivie d'une minimisation de l'énergie par 500 cycles de Powell. Dans cette étape le poids des interactions de Van der Waals est maintenu faible. Jusqu'à présent, un paramétrage simplifié a été utilisé pour générer rapidement des structures compatibles avec les données RMN (par exemple, pendant l'étape de recuit simulé tous les termes d'énergie électrostatique ont été supprimés). La dernière étape de cette modélisation consiste donc à minimiser l'énergie potentielle des différentes structures obtenues.

### EXEMPLE 2 : ANTIGENICITE DU PEPTIDE SEQ ID NO: 2

Les propriétés antigéniques du peptide ont été analysées par la demanderesse par des tests ELISA ci-après décrits, et pratiqués avec des sérums provenant de personnes infectées par le VHC. Pour chacun des dix sérums testés, un puits supplémentaire, dans lequel le peptide n'a pas été déposé, sert de témoin interne.

### a) Protocole des tests ELISA

Cette technique permet de mettre en évidence la présence d'anticorps spécifiques dans le sérum ou le plasma d'un individu et, par extension, la présence de l'antigène correspondant dans son organisme.

Les puits d'une plaque de microtitration de marque "NUNC maxisorb" sont saturés par 100 µl d'une solution tampon carbonate, pH 9,6, contenant le peptide (10 µg/ml) durant 2 heures. La plaque est ensuite vidée puis lavée à l'aide d'un tampon de lavage contenant 0,05 % de Tween 20. Les puits sont saturés par 100 µl de tampon de lavage additionné de 10 % de sérum de chèvre (v/v), incubés pendant 30 minutes à 37°C, puis lavés de nouveau comme précédemment. Les sérums à analyser sont dilués de façon appropriée avec le tampon de saturation, puis incubés dans les puits 1 heure à 37°C. Les puits sont de nouveau lavés. La solution de conjugué (IgG de chèvre anti IgG humain marquées par la peroxydase) à une dilution de 1/1000 dans le tampon de saturation est alors ajoutée. Après 10 minutes, la réaction est bloquée par 50 µl d'H₂SO₄ et la densité optique est lue à 492 nm.

### b) Résultats

Le tableau 1 ci-après regroupe les résultats des tests ELISA, exprimés en densité optique (DO) lue à 492 nm après réaction de la peroxydase. Ce tableau montre que le peptide réagit fortement avec pratiquement tous les sérums contenant des anticorps anti-nucléocapside puisque les densités optiques (DO) observées pour ce peptide sont généralement très supérieures à celles du puits témoin.Le sérum H662 avec lequel le peptide ne réagit pas est de type 1+, c'est-à-dire un sérum répertorié comme peu réactif vis-à-vis de la nucléocapside.

On remarque enfin que le peptide ne présente pas de réaction croisée avec les deux sérums dénommés "neg" qui ne contiennent pas d'anticorps dirigés contre la nucléocapside.

**Tableau 1**

| Sérum | Dilution | Peptide | Témoin |
|---|---|---|---|
| *4+H8 | | 0,097 | 0,025 |
| 4+H69 | 1/1000 | 0,455 | 0,018 |
| 4+H666 | | 0,330 | 0,016 |
| *3+H1 | 1/100 | 0,375 | 0,036 |
| 3+H3 | | 0,726 | 0,070 |
| *2+H654 | | 0,251 | 0,026 |
| *1+H661 | 1/100 | 0,050 | 0,043 |
| 1+H662 | | 0,028 | 0,031 |
| neg H4 | 1/100 | 0,042 | 0,051 |
| neg H647 | | 0,026 | 0,028 |

| | | | |
|---|---|---|---|
| * Les sérums sont classés en fonction de leur réactivité, et sont affectés d'un indice : les sérums d'indice 4+ réagissent fortement, et les sérums d'indice 1+ réagissent faiblement vis-à-vis d'un test de confirmation de référence, par exemple RIBA 3 (commercialisé par ORTHO DIAGNOSTIC). | | | |

### EXEMPLE 3 : CARACTERISATION PAR RMN DE LA STRUCTURE TRIDIMENSIONNELLE D'UN PEPTIDE DE L'INVENTION

Pour des raisons expérimentales, un peptide dénommé Capsid (15-41), plus long que le peptide de référence (SEQ ID NO: 2) précédemment identifié, de deux acides aminés, a été synthétisé puis caractérisé par RMN, selon la procédure et le matériel identifié dans l'exemple 1, en plaçant ledit peptide dans 40 % de TFE / 60 % H₂O, NaCl 0,1 M, phosphate de sodium 10 mM, à pH 5,9 et à 20°C.

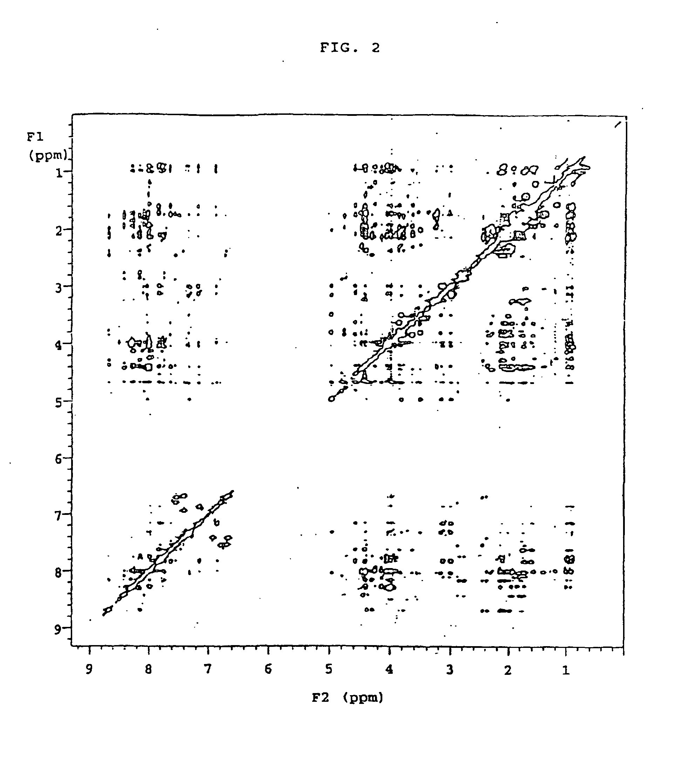

La figure 2 montre que le spectre NOESY du peptide, enregistré avec un temps de mélange de 250 ms, sur 2048 points dans la dimension t1 et 512 dans la dimension t2. En t1 et t2, un zéro filling respectivement à 4 K et 2 K a été appliqué.

Le filtrage est de type sinus carré dans les deux dimensions. Ce spectre est plus particulièrement représentatif de la structure secondaire du peptide considéré.

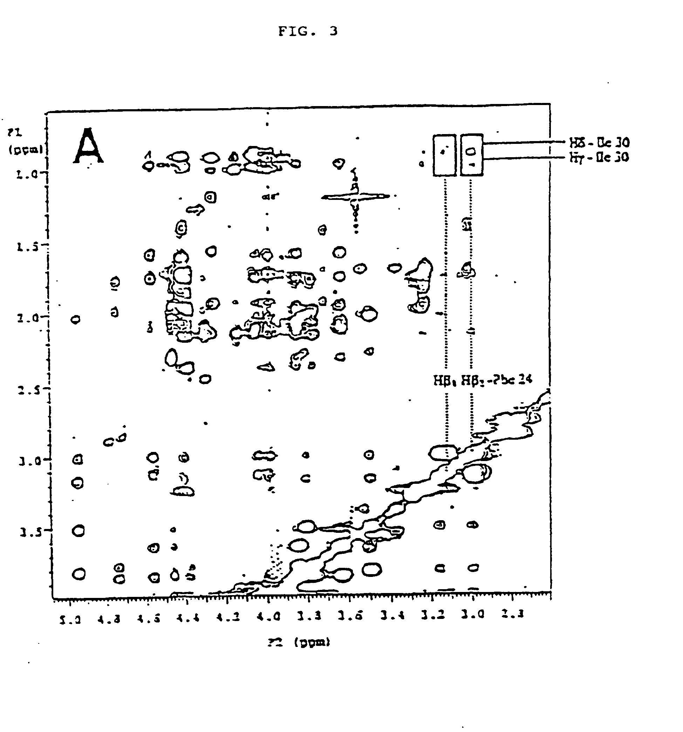

Le spectre selon Fig. 3 a été enregistré avec un temps de mélange de 250 ms. Les corrélations entre les protons β de la Phe 24, et les protons γ et δ de l'Ile 30 ont été indiquées, et sont caractéristiques de la structure tertiaire précédemment définie.

### EXEMPLE 4 : MODELISATION MOLECULAIRE DE LA STRUCTURE TRIDIMENSIONNELLE DU PEPTIDE CAPSID (15-41)

La modélisation moléculaire du peptide Capsid (15-41) a été réalisée à partir de 252 et de 268 contraintes respectivement (Tableau 2 ci-après). Sur les 50 structures générées pour chaque peptide, on obtient 32 structures acceptées pour le peptide Capsid (15-41).

Le modèle de peptide Capsid (15-41) montre que ce peptide contient bien les deux hélices du peptide et que ces deux hélices sont bien maintenues perpendiculaires l'une par rapport à l'autre. On retrouve donc bien, pour ce peptide, le motif 3D hélice-boucle-hélice.

Le tableau 2 donne les contraintes RMN du peptide Capsid (15-41) dans lequel les contraintes intra-résiduelles (i,i) correspondent aux protons portés par un même résidu d'acide aminé ; les contraintes séquentielles (i, i+1) relient des protons situés sur deux résidus adjacents ; les contraintes dites moyenne portée (i, i+2 à i+4) impliquent des protons séparés par au plus quatre résidus dans la séquence ; et les contraintes de type longue portée (i, i>i+4) relient les protons de résidus séparés par plus de 4 acides aminés.

**Tableau 2**

| | |
|---|---|
| Type de contrainte | |
| Intra-résiduelle (i,i) | 70 |
| Séquentielle (i, i+1) | 97 |
| Moyenne (i, i+2 à i+4) | 73 |
| Longue (i, i>i+4) | 12 |
| Total | 252 |

### LISTAGE DES SEQUENCES

### NOMBRE DE SEQUENCES : 10

1) INFORMATION CONCERNANT SEQ ID NO: 1:
   i) CARACTERISTIQUES DE LA SEQUENCE:
      A) LONGUEUR : 44 acides aminés
      B) TYPE : séquence d'acides aminés
   ii) TYPE DE MOLECULE : peptide
   iii) HYPOTHETIQUE : non
   v) TYPE DE FRAGMENT : N-terminal
   vi) ORIGINE :
      A) ORGANISME : virus humain de l'hépatite C
      B) SOUCHE : H77 publiée selon Ogata N. et al, Nucleotide Sequence and Mutation Rate of the H strain of Hepatitis Virus, Proc. Natl. Academ Sci. USA, 88:3392-6 (1991)
   ix) CARACTERISTIQUES :
      A) NOM/CLE : peptide
      B) EMPLACEMENT : 2..45
      D) AUTRES INFORMATIONS :
         séquence N-terminale de la protéine de nucléocapside ou protéine CORE du virus humain de l'hépatite C.
   xi) DESCRIPTION DE LA SEQUENCE : SEQ ID NO: 1: 
Ser Thr Asn Pro Lys Pro Gln Arg Lys Thr Lys Arg Asn Thr Asn Arg
1               5                   10                  15
Arg Pro Gln Asp Val Lys Phe Pro Gly Gly Gly Gln Ile Val Gly Gly
            20                  25                  30
Val Tyr Leu Leu Pro Arg Arg Gly Pro Arg Leu Gly
        35                  40              44
2) INFORMATION CONCERNANT SEQ ID NO: 2:
   i) CARACTERISTIQUES DE LA SEQUENCE:
      A) LONGUEUR : 25 acides aminés
      B) TYPE : séquence d'acides aminés
   ii) TYPE DE MOLECULE : peptide
   iii) HYPOTHETIQUE : non
   xi) DESCRIPTION DE LA SEQUENCE : SEQ ID NO: 2: 
Thr Asn Arg Arg Pro Gln Asp Val Lys Phe Pro Gly Gly Gly Gln Ile
1               5                   10                  15
Val Gly Gly Val Tyr Leu Leu Pro Arg
            20                  25
3) INFORMATION CONCERNANT SEQ ID NO: 3:
   i) CARACTERISTIQUES DE LA SEQUENCE:
      A) LONGUEUR : 25 acides aminés
      B) TYPE : séquence d'acides aminés
   ii) TYPE DE MOLECULE : peptide
   iii) HYPOTHETIQUE : non
   xi) DESCRIPTION DE LA SEQUENCE : SEQ ID NO: 3: 
Thr Tyr Arg Arg Pro Gln Asp Val Lys Phe Pro Gly Gly Gly Gln Ile
1               5                   10                  15
Val Gly Gly Val Tyr Leu Leu Pro Arg
            20                  25
4) INFORMATION CONCERNANT SEQ ID NO: 4:
   i) CARACTERISTIQUES DE LA SEQUENCE:
      A) LONGUEUR : 25 acides aminés
      B) TYPE : séquence d'acides aminés
   ii) TYPE DE MOLECULE : peptide
   iii) HYPOTHETIQUE : non
   xi) DESCRIPTION DE LA SEQUENCE : SEQ ID NO: 4: 
Thr Ile Arg Arg Pro Gln Asp Val Lys Phe Pro Gly Gly Gly Gln Ile
1               5                   10                  15
Val Gly Gly Val Tyr Leu Leu Pro Arg
            20                  25
5) INFORMATION CONCERNANT SEQ ID NO: 5:
   i) CARACTERISTIQUES DE LA SEQUENCE:
      A) LONGUEUR : 25 acides aminés
      B) TYPE : séquence d'acides aminés
   ii) TYPE DE MOLECULE : peptide
   iii) HYPOTHETIQUE : non
   xi) DESCRIPTION DE LA SEQUENCE : SEQ ID NO: 5: 
Thr Asn Arg Arg Pro Met Asp Val Lys Phe Pro Gly Gly Gly Gln Ile
1               5                   10                  15
Val Gly Gly Val Tyr Leu Leu Pro Arg
            20                  25
6) INFORMATION CONCERNANT SEQ ID NO: 6:
   i) CARACTERISTIQUES DE LA SEQUENCE:
      A) LONGUEUR : 25 acides aminés
      B) TYPE : séquence d'acides aminés
   ii) TYPE DE MOLECULE : peptide
   iii) HYPOTHETIQUE : non
   xi) DESCRIPTION DE LA SEQUENCE : SEQ ID NO: 6: 
Thr Asn Arg Arg Pro Gln Asp Val Lys Phe Pro Gly Gly Gly Gln Ile
1               5                   10                  15
Val Gly Gly Val Tyr Val Leu Pro Arg
            20                  25
7) INFORMATION CONCERNANT SEQ ID NO: 7:
   i) CARACTERISTIQUES DE LA SEQUENCE:
      A) LONGUEUR : 25 acides aminés
      B) TYPE : séquence d'acides aminés
   ii) TYPE DE MOLECULE : peptide
   iii) HYPOTHETIQUE : non
   xi) DESCRIPTION DE LA SEQUENCE : SEQ ID NO: 7: 
Thr Tyr Arg Arg Pro Gln Asp Val Lys Phe Pro Gly Gly Gly Gln Ile
1               5                   10                  15
Val Gly Gly Val Tyr Val Leu Pro Arg
            20                  25
8) INFORMATION CONCERNANT SEQ ID NO: 8:
   i) CARACTERISTIQUES DE LA SEQUENCE:
      A) LONGUEUR : 25 acides aminés
      B) TYPE : séquence d'acides aminés
   ii) TYPE DE MOLECULE : peptide
   iii) HYPOTHETIQUE : non
   xi) DESCRIPTION DE LA SEQUENCE : SEQ ID NO: 8: 
Thr Ile Arg Arg Pro Gln Asp Val Lys Phe Pro Gly Gly Gly Gln Ile
1               5                   10                  15
Val Gly Gly Val Tyr Val Leu Pro Arg
            20                  25
9) INFORMATION CONCERNANT SEQ ID NO: 9:
   i) CARACTERISTIQUES DE LA SEQUENCE:
      A) LONGUEUR : 25 acides aminés
      B) TYPE : séquence d'acides aminés
   ii) TYPE DE MOLECULE : peptide
   iii) HYPOTHETIQUE : non
   xi) DESCRIPTION DE LA SEQUENCE : SEQ ID NO: 9: 
Thr Ile Arg Arg Pro Gln Asp Ile Lys Phe Pro Gly Gly Gly Gln Ile
 1               5                  10                  15
Val Gly Gly Val Tyr Ile Leu Pro Arg
            20                  25
10) INFORMATION CONCERNANT SEQ ID NO: 10:
   i) CARACTERISTIQUES DE LA SEQUENCE:
      A) LONGUEUR : 25 acides aminés
      B) TYPE : séquence d'acides aminés
   ii) TYPE DE MOLECULE : peptide
   iii) HYPOTHETIQUE : non
   iv) AUTRE INFORMATION : Xaa est Ile ou Val ou Leu
   xi) DESCRIPTION DE LA SEQUENCE : SEQ ID NO: 10:

Thr Asn Arg Arg Pro Gln Asp Xaa Lys Phe Pro Gly Gly Gly Gln Xaa
1               5                   10                  15
Xaa Gly Gly Xaa Tyr Xaa Xaa Pro Arg
            20                  25

## Revendications

1. Peptide structural, reconnu par les anticorps dirigés contre un polypeptide comprenant la séquence des acides aminés 2-45 de l'extrémité N-terminale de la protéine Core ou nucléocapside (p21) du virus de l'hépatite C (VHC) ou de tout variant dudit virus comprenant une structure tertiaire qui est constituée par au moins un premier fragment peptidique ayant une structure secondaire en hélice α, un second fragment peptidique ayant une structure secondaire en hélice α et un troisième fragment peptidique de jonction reliant les deux hélices α, ces deux hélices a étant sensiblement perpendiculaires l'une par rapport à l'autre, dans l'espace, ledit peptide consistant en la séquence SEQ ID NO : 2 ou en une séquence peptidique équivalente à SEQ ID NO :2 choisie parmi l'une quelconque des séquences SEQ ID NO :3 à SEQ ID NO :10.

2. Composé antigénique, reconnu par les anticorps dirigés contre un polypeptide comprenant la séquence des acides aminés 2-45 de l'extrémité N-terminale de la protéine Core (p21) du virus de l'hépatite C (VHC), **caractérisé en ce qu'**il comprend un peptide selon la revendication 1.

3. Composé immunogène, susceptible d'induire la production d'anticorps dirigés contre un polypeptide comprenant la séquence des acides aminés 2-45 de l'extrémité N-terminale de la protéine Core (p21) du virus de l'hépatite C (VHC), ou de tout variant dudit virus, **caractérisé en ce qu'**il comprend un peptide selon la revendication 1.

4. Utilisation d'un peptide selon la revendication 1, ou d'un composé selon la revendication 2, pour détecter et/ou quantifier des anticorps dirigés contre la protéine Core (p21) du VHC.

5. Utilisation d'un peptide selon la revendication 1, d'un composé selon la revendication 2 ou 3, pour préparer une composition diagnostique, prophylactique ou thérapeutique, apte à détecter, prévenir ou traiter une infection par le VHC, ou tout variant dudit virus.

6. Composition pharmaceutique comprenant un peptide selon la revendication 1, un composé selon la revendication 2 ou 3.

7. Procédé de détection et/ou quantification, dans un échantillon, d'anticorps dirigés contre la protéine Core (p21) du virus de l'hépatite C (VHC), **caractérisé en ce qu'**on met en contact ledit' échantillon avec un peptide selon la revendication 1, et/ou un composé selon la revendication 2, et on détecte la formation d'un complexe immun entre lesdits anticorps et ledit peptide ou composé.

8. Procédé de détection et/ou quantification, dans un échantillon, de tout ou partie de l'ARN du virus de l'hépatite C, **caractérisé en ce qu'**on met en contact ledit échantillon avec un peptide selon la revendication 1, et/ou un composé selon la revendication 2 ou 3, et on détecte la formation d'un complexe entre ledit ARN et ledit peptide ou composé.

9. Complexe comprenant un peptide selon la revendications 1 et une structure moléculaire liée spécifiquement avec ledit peptide, ladite structure moléculaire étant notamment choisie parmi des fragments peptidiques, des fragments nucléotidiques, des molécules chimiques telles que celles du type aromatique fonctionnalisé.

## Claims

1. Structural peptide, recognized by the antibodies directed against a polypeptide comprising the sequence of amino acids 2-45 of the N-terminal end of the Core or nucleocapsid protein (p21) of the hepatitis C virus (HCV) or of any variant of said virus, **characterized in that** it comprises a tertiary structure consisting of at least a first peptide fragment having a secondary structure in the form of an α helix, a second peptide fragment having a secondary structure in the form of an α helix and a third joining peptide fragment linking the two α helices, these two α helices being substantially perpendicular to each other in space, said peptide consisting of the sequence SEQ ID NO: 2 or of a peptide sequence equivalent to SEQ ID NO: 2 chosen from any one of the sequences SEQ ID NO: 3 to SEQ ID NO: 10.

2. Antigenic compound, recognized by antibodies directed against a polypeptide comprising the sequence of amino acids 2-45 of the N-terminal end of the Core protein (p21) of the hepatitis C virus (HCV), **characterized in that** it comprises a peptide according to Claim 1.

3. Immunogenic compound, capable of inducing the production of antibodies directed against a polypeptide comprising the sequence of amino acids 2-45 of the N-terminal end of the Core protein (p21) of the hepatitis C virus (HCV), or of any variant of said virus, **characterized in that** it comprises a peptide according to Claim 1.

4. Use of a peptide according to Claim 1, or of a compound according to Claim 2, for detecting and/or quantifying antibodies directed against the core protein (p21) of HCV.

5. Use of a peptide according to Claim 1, or of a compound according to Claim 2 or 3, for preparing a diagnostic, prophylactic or therapeutic composition capable of detecting, preventing or treating an infection with HCV, or any variant of said virus.

6. Pharmaceutical composition comprising a peptide according to Claim 1 or a compound according to Claim 2 or 3.

7. Method of detecting and/or quantifying, in a sample, antibodies directed against the Core protein (p21) of the hepatitis C virus (HCV), **characterized in that** said sample is brought into contact with a peptide according to Claim 1, and/or a compound according to Claim 2, and the formation of an immune complex between said antibodies and said peptide or compound is detected.

8. Method of detecting and/or quantifying, in a sample, all or part of the RNA of the hepatitis C virus, **characterized in that** said sample is brought into contact with a peptide according to Claim 1, and/or a compound according to Claim 2 or 3, and the formation of a complex between said RNA and said peptide or compound is detected.

9. Complex comprising a peptide according to Claim 1 and a molecular structure specifically bound to said peptide, said molecular structure being in particular chosen from peptide fragments, nucleotide fragments and chemical molecules such as those of the functionalized aromatic type.

## Patentansprüche

1. Strukturpeptid, das von den gegen ein Polypeptid umfassend die Aminosäuresequenz 2-45 des N-terminalen Endes des Core-Proteins oder Nukleocapsids (p21) des Hepatitis-C-Virus (HCV) oder jeglicher Variante dieses Virus gerichteten Antikörpern erkannt wird und das eine Tertiärstruktur umfaßt, die aus mindestens einem ersten Peptidfragment mit einer α-Helix-Sekundärstruktur, einem zweiten Peptidfragment mit einer α-Helix-Sekundärstruktur und einem dritten Peptidfragment als Linker, das die zwei α-Helices verbindet, besteht, wobei die beiden α-Helices räumlich im wesentlichen rechtwinklig zueinander angeordnet sind, wobei das Peptid aus der Sequenz SEQ ID Nr. 2 oder aus einer zu SEQ ID Nr. 2 äquivalenten Peptidsequenz, die unter einer der Sequenzen SEQ ID Nr. 3 bis SEQ ID Nr. 10 ausgewählt ist, besteht.

2. Antigene Verbindung, die von den gegen ein Polypeptid umfassend die Aminosäuresequenz 2-45 des N-terminalen Endes des Core-Proteins (p21) des Hepatitis-C-Virus (HCV) gerichteten Antikörpern erkannt wird, **dadurch gekennzeichnet, daß** sie ein Peptid nach Anspruch 1 umfaßt.

3. Immunogene Verbindung, die fähig ist, die Produktion von gegen ein Polypeptid umfassend die Aminosäuresequenz 2-45 des N-terminalen Endes des Core-Proteins (p21) des Hepatitis-C-Virus (HCV) oder jeglicher Variante dieses Virus gerichteten Antikörpern zu induzieren, **dadurch gekennzeichnet, daß** sie ein Peptid nach Anspruch 1 umfaßt.

4. Verwendung eines Peptids nach Anspruch 1 oder einer Verbindung nach Anspruch 2 für den Nachweis und/oder die quantitative Bestimmung von gegen das Core-Protein (p21) des HCV gerichteten Antikörpern.

5. Verwendung eines Peptids nach Anspruch 1 oder einer Verbindung nach Anspruch 2 oder 3 zur Herstellung einer diagnostischen, prophylaktischen oder therapeutischen Zusammensetzung, die sich für den Nachweis, die Vorbeugung gegen oder die Behandlung einer Infektion durch HCV oder jegliche Variante dieses Virus eignet.

6. Pharmazeutische Zusammensetzung, umfassend ein Peptid nach Anspruch 1 oder eine Verbindung nach Anspruch 2 oder 3.

7. Verfahren für den Nachweis und/oder die quantitative Bestimmung von gegen das Core-Protein (p21) des Hepatitis-C-Virus (HCV) gerichteten Antikörpern in einer Probe, **dadurch gekennzeichnet, daß** man die Probe mit einem Peptid nach Anspruch 1 und/oder einer Verbindung nach Anspruch 2 in Kontakt bringt und die Bildung eines Immunkomplexes zwischen den Antikörpern und dem Peptid bzw. der Verbindung nachweist.

8. Verfahren für den Nachweis und/oder die quantitative Bestimmung der Gesamt-RNA oder eines Teils der RNA des Hepatitis-C-Virus in einer Probe, **dadurch gekennzeichnet, daß** man die Probe mit einem Peptid nach Anspruch 1 und/oder einer Verbindung nach Anspruch 2 oder 3 in Kontakt bringt und die Bildung eines Komplexes zwischen dieser RNA und dem Peptid bzw. der Verbindung nachweist.

9. Komplex umfassend ein Peptid nach Anspruch 1 und eine Molekülstruktur, die spezifisch mit diesem peptid bindet, wobei die Molekülstruktur insbesondere aus der Reihe Peptidfragmente, Nukleotidfragments oder Chemikalienmoleküle wie solche des funktionalisierten Aromatentyps stammt.
